# EUROPEAN PATENT APPLICATION

(11) **EP 4 159 284 A1**
(43) Date of publication of application: **05.04.2023**
(21) Application number: 21813509.3
(22) Date of filing: 21.05.2021
(51) Int. Cl.: A61Q 19/08, A61K 8/66

(54) **ELASTIN PRODUCTION PROMOTER AND COSMETIC PREPARATION FOR SKIN**

(30) Priority: 25.05.2020 JP 2020090313
(71) Applicant: Shizuoka Prefectural University Corporation, Shizuoka-shi, Shizuoka 422-8526 (JP); Nikken Foods Company Limited, Fukuroi-shi, Shizuoka 437-0122 (JP)
(72) Inventor: MINAMI, Akira, Shizuoka-shi, Shizuoka 422-8526 (JP); SUZUKI, Takashi, Shizuoka-shi, Shizuoka 422-8526 (JP); IWAO, Yasunori, Shizuoka-shi, Shizuoka 422-8526 (JP)
(74) Representative: Mewburn Ellis LLP
(86) International application number: PCT/JP2021/019322
(87) International publication number: WO 2021/241428

(57) **Abstract**

The present invention provides a novel elastin production promoter. The elastin production promoter contains sialidase as an active ingredient.

## Description

### Technical Field

The present invention relates to elastin production promoters, and, in particular, to an elastin production promoter capable of increasing dermal elastin decreased with age.

### Background Art

The skin consists of the epidermis, the dermis, and the subcutis. Of these, in the dermis present underneath the epidermis, collagen fibers imparting the strength to the skin and elastin supporting the collagen fibers are distributed. Elastin is elastic fibers that have elasticity and stretchability, and acts to impart elasticity and firmness to the skin. However, elastin decreases in amount with age and is known to be one of the causes for wrinkles and sagging of the skin.

For this reason, various elastin production promoters have been proposed to prevent wrinkles and sagging of the skin. For example, Patent Literature 1 describes an elastin production promoter having, as an active ingredient, lipocalin which is a protein belonging to the lipocalin family or a decomposition product thereof, and Patent Literature 2 describes an elastin production promoter having a specific dipeptide or tripeptide as an active ingredient.

On the other hand, a sialidase is a hydrolase that cleaves sialic acid from a glycan. Sialic acid mainly modifies a glycan terminus and is involved with a wide range of vital phenomena such as physiological functions, diseases, and virus infections. Thus, sialidases are known to play a role in controlling cell functions such as cell differentiation cell proliferation, and apoptosis. For this reason, the present inventors have developed a fluorescence imaging probe capable of visualizing the enzyme activity of sialidases on tissues with high sensitivity to pursue knowledges on sialidases responsible for a key role within an organism (Non Patent Literature 1).

The present inventors have been figuring out new physiological roles of sialidases using the fluorescence imaging probe described above. As an example, Patent Literature 3 reports new findings on the detection of sialidase activity in the pancreatic islets, as well as a suppressing effect on blood glucose level elevation and an increasing action on insulin secretion level by a compound having sialidase inhibitory activity.

Sialidases roughly have 3 categories of animal (mammals) sialidases, viral sialidases, and bacterial sialidases. Of these, sialidase NEU1, which is a type of animal sialidase, is documented to form a molecular complex with an elastin binding protein (EBP) and cathepsin A and be involved with the formation of an elastin fiber assembly, that is, elastic fibers (Non Patent Literature 2).

### Citation List

### Patent Literature

Patent Literature 1
   Japanese Patent Laid-Open No. 2015-000860
Patent Literature 2
   Japanese Patent Laid-Open No. 2014-141450
Patent Literature 3
   Japanese Patent Laid-Open No. 2017-222641

### Non Patent Literature

### Non Patent Literature 1

Minami A. et al., PLOS ONE, Volume 9, Issue 1, e81941, 2014 Non Patent Literature 2
Hinek A. et al., J. Biol. Chem., Vol.281, No.6, pp.3698-3710, 2006

### Summary of Invention

### Technical Problem

However, there has been no report so far on the visualization of the sialidase activity distribution in the skin tissues using the fluorescence imaging probe as reported in Non Patent Literature 1. There has also been no report on studies on the relevance between sialidases and the phenomenon where dermal elastin decreases in amount with age.

Further, a new material having an elastin production promoting effect is still expected to be developed and provided as anti-aging awareness increases, however, no studies have been conducted so far on the use of a sialidase for promoting elastin production in the skin tissues, and effectiveness thereof has been completely unknown.

Accordingly, the present invention has been accomplished in light of the aspect described above, and an object thereof is to provide a novel elastin production promoter.

Additionally, another object of the present invention is to provide an elastin production promoter capable of ameliorating or preventing the decrease of dermal elastin with age.

### Solution to Problem

The present inventors were pursuing the involvement of sialidases in dementia using the fluorescence imaging probe described in Non Patent Literature 1, in the process of which the inventors incidentally found that sialidase activity in the skin tissues notably reduced with aging. Based on this finding, the present invention has come to accomplishment.

For solving the above problem, the elastin production promoter of the present invention contains a sialidase as an active ingredient. Sialidase administration to target tissues can promote elastin production and increase an elastin level in the target tissues.

In the elastin production promoter of the present invention, the sialidase described above is also preferably sialidase NEU2. This enables the selection of a preferable type of sialidase as an elastin production promoter.

Further, in the elastin production promoter of the present invention, the sialidase described above is also preferably a bacterial sialidase. This enables the selection of a preferable type of sialidase as an elastin production promoter. Additionally, bacterial sialidases have high sialidase activity and excellent stability, thereby being easy to handle.

In the elastin production promoter of the present invention, the bacterial sialidase described above is also preferably a sialidase derived from a bacterium of the genus *Arthrobacter (Arthrobacter* sp.). This enables the selection of a more preferable type of sialidase that has high sialidase activity and excellent safety and stability.

For the elastin production promoter of the present invention, the elastin described above is also preferably dermal elastin constituting fibrous tissues of the dermis. This enables the selection of a suitable target in which elastin production is promoted.

The elastin production promoter of the present invention is also preferably for preventing or ameliorating skin aging. Elastin present in the dermis decreases in amount with age and causes wrinkles and sagging of the skin, but sialidase administration promotes elastin production and increases an elastin level. For this reason, firmness and elasticity of the skin can be retained or restored, thereby preventing or ameliorating skin aging.

The cosmetic preparation for skin of the present invention contains the elastin production promoter described above. This enables to obtain the cosmetic preparation for skin having an action to enhance firmness and elasticity of the skin.

### Advantageous Effects of Invention

According to the present invention, the elastin production promoter and the cosmetic preparation for skin having the following excellent effects can be provided.
(1) Elastin production can be enhanced, and an elastin level in target tissues can be increased.
(2) Dermal elastin can be increased in amount, and thereby firmness and elasticity of the skin can be enhanced.
(3) Decrease of the dermal elastin with aging can be prevented or ameliorated.

### Brief Description of Drawings

[Figure 1] Figure 1 is a fluorescence-imaged image showing sialidase activity of a rat skin tissue section and an H&E-stained image of the same section in Example 1.
[Figure 2] Figure 2 is a graph showing the relation of rat's age in weeks to sialidase activity in the rat skin tissues in Example 2. In the graph, ***: P<0.001 vs. E19, and **†††:** P<0.001 vs. 12 weeks.
[Figure 3] Figure 3 is graphs showing expression ratio of each isozyme of an animal sialidase of the dermis and muscle tissues (Dermis + Muscle) in rat subcutaneous adipose tissues (Fat) in Example 3. The graphs show expression levels of Figure 3(a): sialidase Neu1, Figure 3(b): sialidase Neu2, Figure 3(c): sialidase Neu3, and Figure 3(d): sialidase Neu4. In the graphs, *: P<0.05, and ***: P<0.001.
[Figure 4] Figure 4 is a graph showing elastin production promoting effects on the rat skin when sialidase NEU2 is applied in Example 4. In the graph, **: P<0.01 vs. CAGE, and ***: P<0.001 vs. CAGE.
[Figure 5] Figure 5 is a graph showing mRNA expression levels of the elastin gene when sialidase NEU2 is applied to the rat skin.
[Figure 6] Figure 6 shows Figure 6(b): a graph showing changes in sialidase activity, and Figure 6(c): a graph showing elastin production promoting effects when a bacterial sialidase is applied to the rat skin in Example 5. Additionally, Figure 6(a) is a graph showing the sialidase activities when the bacterial sialidase is mixed respectively with PBS and choline and geranic acid (CAGE), which is an ionic liquid. In the graphs, *: P<0.05 vs. CAGE, and **: P<0.01 vs. CAGE.
[Figure 7] Figure 7 is fluorescence imaging images showing a sialidase activity distribution in a skin section of each test group in Example 5.
[Figure 8] Figure 8 is immunohistochemical stained images showing an elastin distribution in a dermis tissue section of each test group in Example 5.
[Figure 9] Figure 9 is an autofluorescence elastin image showing an elastin distribution in a dermis tissue section of each test group in Example 5. A scale bar shows 50 µm.
[Figure 10] Figure 10 is graphs showing elastin production promoting effects when sialidase is applied to the skin of senescence-accelerated model mice in Example 6. Figure 10(a) shows data of a test group in which SAMP1 was used as the senescence-accelerated model mouse, and Figure 10(b) shows data of a test group in which SAMP8 was used as the senescence-accelerated model mouse. In the graphs, **†††:** P<0.001.
[Figure 11] Figure 11 is a graph showing enzyme activities and durabilities of sialidase NEU1 and sialidase NEU2 in Example 7.
[Figure 12] Figure 12 is a graph showing temporal stability of sialidase NEU1 in Example 7.

### Description of Embodiments

Sialidase (also called as neuraminidase) in the present invention is a hydrolase that releases sialic acid from a glycan, and the sialidase activity refers to the activity capable of releasing sialic acid from a glycan. The sialidase activity can be detected by using, for example, 4MU-Neu5Ac and BTP-Neu5Ac developed by the present inventors, but not limited thereto.

The sialidases used in the present invention are not particularly limited as long as they have the elastin production promoting action, but animal (mammalian) sialidases or bacterial sialidases are preferably used from the viewpoint of excellent elastin production promoting effect. Of these, animal sialidases have four isozymes, NEU1, NEU2, NEU3, and NEU4, and only one isozyme can be used, or two or more can also be used in combination. Further, in the present invention, sialidase NEU2 is particularly preferably used which has been recently found to be expressed at a high level in the dermis (see an Example described later). Sialidase NEU2 is a protein encoded by 380 amino acid residues, and has a molecular weight of about 42 kDa, with optimum pH of near neutral. Additionally, sialidase NEU2 has excellent durability and stably retains enzyme activity, thereby being easy to handle. Only one type of animal sialidase can be used, or two or more types can also be used in combination.

Bacterial sialidases, when compared to animal sialidases, have higher sialidase activity and enzyme stability and are overexpressed in *E. coli,* thereby advantageously being easy to handle. Examples of bacterial sialidase include sialidases derived from bacteria of the genus *Arthrobacter (Arthrobacter* sp.), bacteria of the genus *Bifidobacterium (Bifidobacterium* sp.), bacteria of the genus *Streptococcus (Streptococcus* sp.), *Vibrio cholerae, Salmonella Typhimurium* and *Clostridium perfringens,* and from viewpoints of the elastin production promoting effect, safety and the like, the sialidase derived from *Arthrobacter ureafaciens* of the genus *Arthrobacter* (commercial product: NACALAI TESQUE, INC., model number 24229), the sialidase derived from *Bifidobacterium bifidum* of the genus *Bifidobacterium* and the like can be particularly preferably used. Further, examples of usable viral sialidase include influenza A virus neuraminidase (NA), influenza B virus neuraminidase (NA), and bacteriophage. Only one type of sialidase can be used, or two or more types can also be used in combination.

The sialidases in the present invention can be obtained by protein expression by gene recombination using the gene encoding a sialidase in accordance with routine procedures. Specifically, a DNA fragment encoding a sialidase as an insert is inserted downstream of a promoter of a suitable expression vector to obtain a recombinant vector. The promotor is a DNA sequence showing transcription activation in a host cell and can suitably be selected in accordance with the type of host. The introduction of this recombinant vector into a suitable host cell provides a transformant that produces a protein having sialidase activity. The transformed host cell is cultured and induced to express a recombinant protein, whereby the protein is produced in cultured cells or cultures. The produced protein can be recovered by a predetermined method. In addition to the protein expression by gene recombination described above, a sialidase can also be obtained by extracting and purifying cells or tissues containing the sialidase.

The elastin production promoter of the present invention contains the sialidase described earlier as the active ingredient and has an action to increase an elastin level in tissues containing elastin. Examples of tissues containing elastin include skin dermis, blood vessel wall, ligament, and lung tissue, however, in the present invention, the elastin production promoter particularly has the action to increase dermal elastin contained in the skin dermis and constituting fibrous tissues. Elastin present in the dermis decreases in amount with age and causes wrinkles and sagging of the skin, but administration of the sialidase described above promotes elastin production and increases an elastin level in the dermis even in the dermis in which an elastin level has once decreased. For this reason, the elastin production promoter of the present invention can retain or restore firmness and elasticity of the skin, thereby preventing or ameliorating skin aging.

The dose of the elastin production promoter of the present invention cannot be decisively specified because it varies depending on an intended ameliorating or preventing effect, age, administration method and the like, however, the typical daily dose in term of a sialidase, i.e., the active ingredient, is preferably about 0.0001 U to 10 U, more preferably about 0.001 U to 1 U, and more preferably about 0.005 U to 0.5 U. In further detail description, the transdermal dose per square centimeter of an application area in term of a sialidase, i.e., the active ingredient, is, for example, preferably about 0.00005 U to 5 U, more preferably about 0.0005 U to 0.5 U, and more preferably about 0.0025 U to 0.25 U.

When the elastin production promoter of the present invention is transdermally administered, a sialidase, i.e., the active ingredient, must be allowed to penetrate inside skin tissues. For this reason, it is preferable to use a substance that has a transdermal drug delivery action when administering the elastin production promoter of the present invention. The substance that has a transdermal drug delivery action can be any substance as long as it does not affect the actions and effects of the present invention, and existing TDDS (Transdermal Drug Delivery System) can be used. In more detail, it is preferable to use an organic salt that is liquid at ordinary temperatures and normal pressures, so called "an ionic liquid" for the transdermal drug delivery. The ionic liquid preferably uses choline as a cation, and examples include choline and geranic acid, choline oleic acid, and choline malonic acid, of which, choline and geranic acid (CAGE) represented by the following Formula 1 can be particularly preferably used.

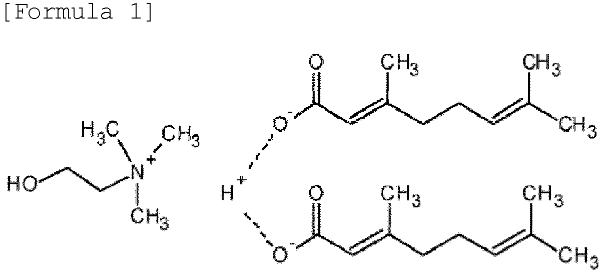

The substance that has a transdermal drug delivery action as described above can be contained as a mixing ingredient in the elastin production promoter together with a sialidase, i.e., the active ingredient. Thus, a one-agent type elastin promoter can be obtained.

Alternatively, the substance that has a transdermal drug delivery action as described above can also be administered separately from a sialidase, i.e., the active ingredient. In this case, the elastin production promoter containing a sialidase, i.e., the active ingredient, is applied to the skin, and then a substance that has a transdermal drug delivery action such as CAGE is applied over the sialidase that has been applied. Example 4 described later shows that such twice-application administration is also effective.

Further, the TDDS (Transdermal Drug Delivery System) described above can also be performed by physical transdermal drug delivery using a microneedle, a needless injector, iontophoresis, phonophoresis, electroporation or the like, in addition to the drug delivery by a chemical substance.

The elastin production promoter of the present invention can also be used as a cosmetic preparation for skin having an anti-aging effect. This retains or restores firmness and elasticity of the skin, and prevents or ameliorates wrinkles and sagging of the skin.

The elastin production promoter or the cosmetic preparation for skin of the present invention can contain various components typically contained in external agents within the range of not affecting the actions and effects of the present invention. Examples include a moisturizer, an emollient agent, a plant extract, a plant fat/oil, a pH regulator, a surfactant, a thickener, vitamins, amino acids, a preservative, an antibacterial agent, a perfume, and a pigment.

Further, the elastin production promoter or the cosmetic preparation for skin of the present invention is applicable for dosage forms of various external agents by a conventional routine method as the typical dosage applied to the skin, and examples include liquid agents such as a lotion, an emulsion, a gel, powders such as a dry water, and a cream. Additionally, the elastin production promoter or the cosmetic preparation for skin can also be applicable for a patch, a pack agent, a makeup product, a cleansing agent, a soap, or a hair care agent and a hair growth formula. When a sialidase, i.e., the active ingredient, and the substance having a transdermal drug delivery action are separately formulated, the separately formulated sialidase and substance can be administered in mixture when used, or the separately formulated sialidase and substance can also be administered simultaneously or at different times.

Hereinafter, the present invention will be described in further detail in reference to Examples but is not at all limited to these Examples.

### Examples

### [Example 1]

### 1. Study on sialidase activity in skin tissues (1)

Presence or absence and distribution of sialidase activity in rat skin tissues were studied using the fluorescence imaging probe (Non Patent Literature 1) developed by the present inventors. This fluorescence imaging probe is BTP3-Neu5Ac in which a molecular species of sialic acid Neu5Ac (N-acetylneuraminic acid) recognized by sialidase is bonded to a phenolic hydroxyl group of a fluorescent substance BTP3[(2-benzothiazol-2-yl)-4-bromophenol]. BTP3-Neu5Ac is soluble in water, and the fluorescence is also off-controlled, but when reacted to a sialidase, water-insoluble fluorescent substance BTB3 is generated and deposits in tissues, and is thereby fluorescence-stained. Thus, sites where sialidase activities are present can histochemically be fluorescence-imaged.

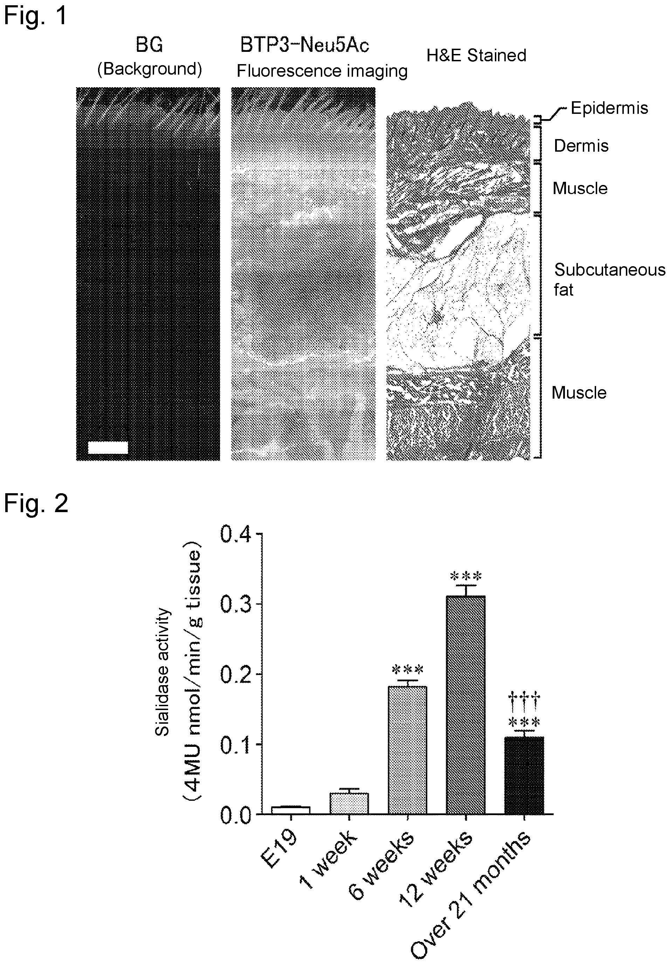

Dorsal skin of a Wistar male rat (12 weeks of age, Japan SLC, Inc.) was embedded using an embedding medium for frozen tissue section preparation (Tissue-Tek (registered trademark) O.C.T. Compound, Sakura Finetek Japan Co., Ltd.). After tissues were frozen, a frozen tissue section having a thickness of 100 to 300 µm was prepared in a cryostat (Leica Microsystems). This section was immersed in 50 to 100 µL of PBS (pH 7.3) containing 1 mM BTP3-Neu5Ac, then washed with PBS, and observed using a fluorescence microscope (Ex/Em = 372 nm/526 nm). The observed section was hematoxylin and eosin stained (H&E staining) and observed. The results are shown in Figure 1.

As shown in Figure 1, in the rat skin tissues, intense fluorescence was detected in a lower part of the dermis. Thus, it was revealed that the lower part of the dermis in the skin tissues had extremely intense sialidase activity.

### [Example 2]

### 2. Study on sialidase activity in skin tissues (2)

The relation of sialidase activity in rat skin tissues to aging was studied. Sialidase activity in the skin of Wistar male rats (Japan SLC, Inc.) at gestation (viviparity) day 19 (E19), 1 week of age, 6 weeks of age, 12 weeks of age, and more than 21 months of age was each measured. Two times the amount of 0.32 M sucrose was added to the collected skin of each rat and the skin was homogenized at 200 rpm while cooled with ice. To the homogenate, PBS (pH 7.3) containing 40 µM 4MU-Neu5Ac (4-methylumbelliferyl-N-acetylneuraminic acid), which is a matrix capable of fluorescence detecting sialidase activity, was added and incubated at 27°C for 1 hour. After terminating the reaction, the fluorescence intensity of 4MU (4-methylumbelliferone) released by the reaction to the sialidase was measured (Ex/Em = 355 nm/460 nm) using a plate reader (Infinite (registered trademark) M200, Tecan Japan Co., Ltd.). The results are shown in Figure 2.

Figure 2 revealed that the sialidase activity increased up to 12 weeks of age, but the rat at more than 21 months of age had the activity decreased to about 1/3 of the rat at 12 weeks of age. Thus, it was the first time to find the notable decrease of the sialidase activity in the skin tissues with age. Based on this finding, the decrease in amount of elastin with age may be involved with the sialidase activity that decreases with age. Accordingly, it is considered that when a decreased sialidase with ageing in the lower layer of the dermis is supplied, there is a potential to suppress the decrease in amount of elastin in the dermis and to promote elastin production in the dermis.

### [Example 3]

### 3. Study on isozymes of sialidases present in the dermis

Animal sialidases have 4 types of isozymes, NEU1, NEU2, NEU3, and NEU4. Four types of isozymes have different locations and substrate specificities respectively, and hence have different functions and roles. In Example 1, as the intense sialidase activity was detected in the dermis lower layer of rat skin tissues, studies were conducted on isozymes of sialidases overexpressed in the dermis.

From Wistar male rat (12 weeks of age, Japan SLC, Inc.), (A) dermis and muscle tissues near the dermis (Dermis + Muscle) and (B) subcutaneous adipose tissues (Fat) were removed respectively. Total RNA was isolated from the removed tissues (A) and (B), respectively. The total RNA was isolated specifically as follows. The tissues were homogenized by adding 1 mL of TRIzol (registered trademark) Reagent (Invitrogen) to the removed tissues respectively and allowed to stand for 5 minutes at room temperature. Subsequently, 100 µL of chloroform was added and mixed by inverting, and then allowed to stand for 3 minutes at room temperature. After left to stand, the tissues were centrifuged at 4°C, 15,000 rpm for 20 minutes to separate the aqueous layer. 250 µL of 2-propanol was added to 250 µL of the aqueous layer, mixed by inverting, and then allowed to stand for 10 minutes at room temperature. After left to stand, the mixture was centrifuged at 4°C, 15,000 rpm for 10 minutes, and after removing the supernatant, 500 µL of 75% ethanol was added and centrifuged at 4°C, 15,000 rpm for 20 minutes. After the supernatant was removed, air drying was performed for 3 minutes, and 30 µL of UltraPure DNase/RNase-Free Distilled Water (Invitrogen) was added. The isolated total RNA was quantitatively measured using a spectrophotometer (NanoDrop ND-1000, Thermo Fisher Scientific), and the concentration of the total RNA was adjusted to 100 ng/µL with UltraPure DNase/RNase-Free Distilled Water.

Using each RNA sample isolated from the removed tissues (A) and (B), mRNA expression levels of 4 types of isozymes of sialidases, that is, the rat sialidases Neu1, Neu2, Neu3, and Neu4 were measured by the real-time RT-PCR method. Specifically, the measurement was performed using One Step TB Green PrimeScript PLUS RT-PCR Kit (Takara Bio Inc.) in accordance with the protocol, and the expression levels of all mRNA were compared. Thermal Cycler Dice (registered trademark) Real Time System Lite (Takara Bio Inc.) was used as the measuring device.

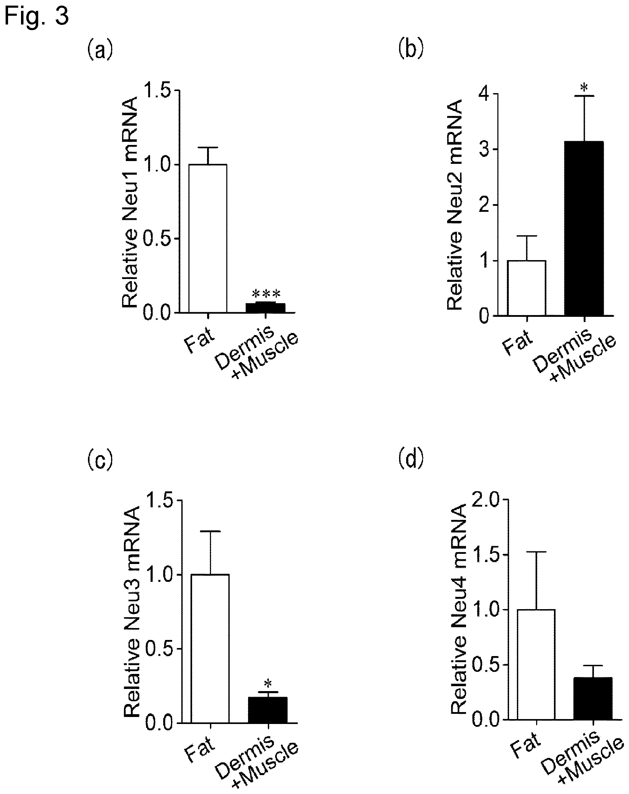

The results are shown in Figure 3. Figure 3(a) shows the mRNA expression level of sialidase Neu1, Figure 3(b) shows the mRNA expression level of sialidase Neu2, Figure 3(c) shows the mRNA expression level of sialidase Neu3, and Figure 3(d) shows the mRNA expression level of sialidase Neu4. Figure 3 shows the mRNA expression level in the dermis and muscle tissues immediately near the lower layer of the dermis (Dermis + Muscle) when the mRNA expression level of subcutaneous adipose tissues (Fat) was 1. The results revealed that, of 4 types of isozymes, sialidase NEU2 was overexpressed in the dermis.

### [Example 4]

### 4. Elastin production promoting effect by sialidase NEU2

Since it was found that the sialidase present in a large amount in the dermis was sialidase NEU2, in the present Example, the elastin production promoting effect by sialidase NEU2 was studied. Specifically, sialidase NEU2 was prepared by a recombinant protein expression in cultured cells, applied to the rat skin for a predetermine period of time, and then an elastin level in the rat skin tissues was measured.

First, a DNA fragment of the gene (2531bp) encoding the rat sialidase NEU2 was prepared by the cloning method. The prepared DNA fragment of the rat Neu2 gene was cloned into a vector as an insert thereby to obtain a recombinant vector containing the Neu2 gene. The constructed NEU2 expression vector was introduced into a C6 glioma cell to perform transfection thereby to obtain NEU2 producing cells. The obtained NEU2 producing cells were cultured in MEM medium to express a protein. The expressed NEU2 protein was recovered from the medium and concentrated by ultrafiltration. To the concentrate, PBS (pH 7.3) containing 4MU-Neu5Ac was added and incubated at 27°C for 1 hour. After terminating the reaction, the fluorescence intensity of 4MU released by the reaction to the sialidase was measured using a plate reader thereby to obtain the sialidase activity (Ex/Em = 355 nm/460 nm). The enzyme activity of the sialidase NEU2 obtained by the recombinant protein expression was 21.3 4MU nmol/min/mL.

The stratum corneum, the outermost layer of the skin cells, has the function of physical barrier for preventing any foreign matter from getting inside. For this reason, even when a protein such as sialidase NEU2 is applied to the skin surface, it is difficult for the stratum corneum to be allowed to penetrate and act in the skin tissues. For this reason, when the sialidase NEU2 obtained as described above was transdermally administered, choline and geranic acid (CAGE), which is an ionic liquid, was used in the present Example. The structural formula of CAGE is shown in the following Formula 2. CAGE is known to be excellent in drug solubility and have a transdermal drug delivery action (see Japanese Translation of PCT International Application Publication No. 2016-535781). In the present Example, a sample solution in which CAGE and sialidase NEU2 were mixed (CAGE + Neu2) was prepared to perform an application test. Additionally, a twice-application test in which a specific amount of sialidase NEU2 was applied followed by applying CAGE (Neu2, CAGE) was also performed. For a comparison control, another test was also performed by applying PBS (phosphate buffer solution) and CAGE, respectively.

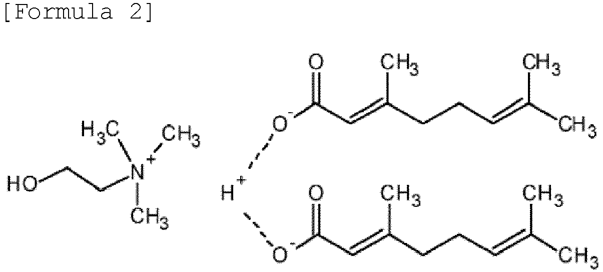

Dorsal hair on the left and right of a Wistar male rat (12 weeks of age, Japan SLC, Inc.) was removed to a size of circle having a diameter of 15 mm to provide sites to which the sample solution was applied. Using each matter shown in the following Table 1, each sample solution was applied to the circular area having a diameter of 15 mm at which rat's hair was removed. The application was performed twice a day for 5 days (total 10 applications).

**[Table 1]**

| Test group | Applied matter |
|---|---|
| PBS | 60 µL of PBS was applied |
| CAGE | 60 µL of CAGE was applied |
| CAGE+Neu2 | 60 µL of mixed solution of 42 µL of CAGE and 18 µL of Neu2 was applied |
| Neu2, CAGE | 18 µL of NEU2 was applied, and then 42 µL of CAGE was applied |

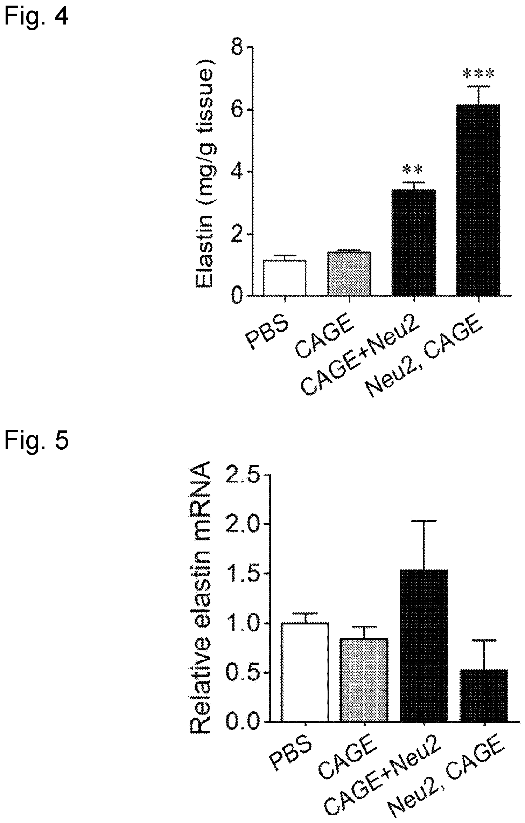

On the following day from the last application, the skin tissues to which the sample solution was applied were collected. The collected skin tissues were homogenized, and then measured for an elastin level in the homogenate of the skin tissues using an elastin colorimetric kit (Fastin Elastin Assay Kit, a product of Biocolor Ltd.). The results are shown in the graph of Figure 4. The results revealed that the elastin level in the skin tissues increased in both of the case in which sialidase NEU2 and CAGE were applied as the mixed solution (CAGE + Neu2) and the case in which sialidase NEU2 was applied followed by applying CAGE (Neu2, CAGE). This revealed that the elastin production in the skin tissues was promoted by transdermal administration of sialidase NEU2.

Further, as shown in the graph of Figure 4, the case in which sialidase NEU2 was applied followed by applying CAGE (Neu2, CAGE) had a large increase of the elastin level, thereby revealing that CAGE was capable of quickly taking in the sialidase NEU2 applied earlier on the skin and delivering into the skin.

Further, the sialidase NEU2 administration was investigated for the impact on mRNA expression of the elastin gene. Specifically, on the following day from the last application of each sample solution shown in Table 1, tissues of the skin, subcutaneous fat and the like on the rat flank were collected, and total RNA was isolated from the rat skin using TRIzol (registered trademark) Reagent (Invitrogen) by the same method as in Example 3. Using the isolated RNA samples, mRNA expression levels of rat elastin gene were measured by the real-time RT-PCR method. The real-time RT-PCR was performed using One-Step SYBR PrimeScript PLUS RT-PCR kit (Takara Bio Inc.) and primers in accordance with the protocol. Thermal Cycler Dice (registered trademark) Real Time System Lite (Takara Bio Inc.) was used as the measuring device. The expression level of elastin mRNA was determined in terms of the relative level of elastin mRNA to the internal standard GAPDH (glyceraldehyde-3-phosphate dehydrogenase). The results are shown in the graph of Figure 5.

As shown in the graph of Figure 5, no statistically significant impact on the mRNA expression level of the elastin gene was found by the sialidase NEU2 administration between the control plot in which the sialidase NEU2 was not administered (PBS, CAGE) and the test plots in which the sialidase NEU2 was administered (CAGE + Neu2, Neu2, CAGE). This showed that the sialidase NEU2 administration had no impact of the mRNA expression level of the elastin gene. As shown in Figure 4, the elastin level in the skin tissues notably increased by the sialidase NEU2 administration, and the elastin production was promoted, and therefore it was presumed that sialidase NEU2 promotes the elastin production by a mechanism other than expression enhancement of the elastin gene.

### [Example 5]

### 5. Elastin production promoting effect by bacterium sialidase

Sialidases have two or more types such as animal sialidases present in mammals, viral sialidases present in viruses, and bacterial sialidases present in bacteria. Of these, a bacterial sialidase having higher sialidases activity than animal sialidases was studied on the elastin production promoting effect.

Specifically, a sialidase derived from *Arthrobacter ureafaciens,* a bacterium of the genus *Arthrobacter,* was applied to the rat's skin for a predetermined period of time, and then an elastin level and sialidase activity in the skin tissues of the rat were measured. In the present Example, a sample solution in which choline and geranic (CAGE) and the sialidase derived from *Arthrobacter ureafaciens* (AUSA) were mixed in specific amounts (CAGE + AUSA) was prepared in the same manner as in Example 4 to perform an application test. For a comparison control, another test was also performed by applying PBS (phosphate buffer solution) and CAGE respectively as the sample solutions.

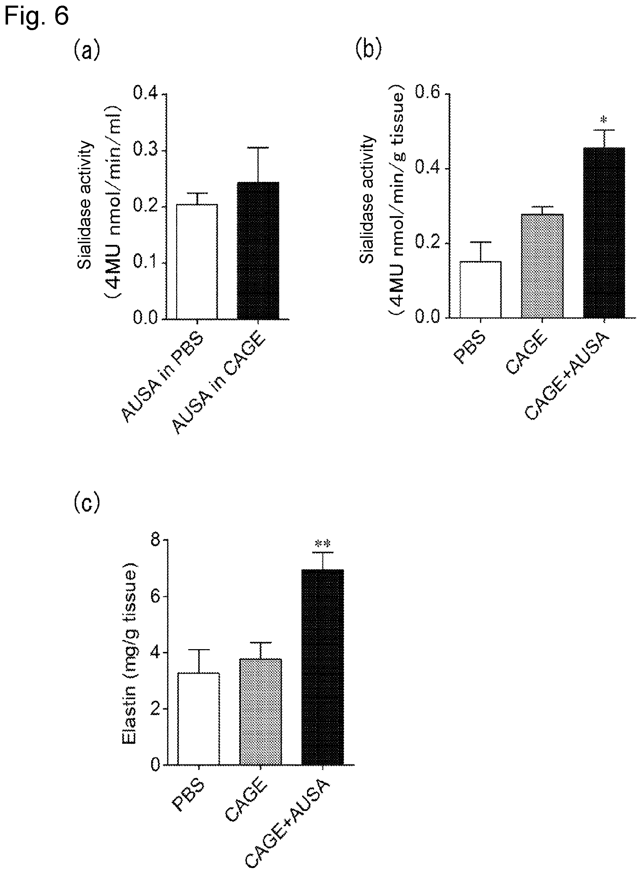

When the sialidase (AUSA) and CAGE were mixed in advance, the retainability of sialidase activity was verified. A sialidase derived from *Arthrobacter ureafaciens (Neuraminidase* from *Arthrobacter ureafaciens,* highly purified, Model number: 24229-74, NACALAI TESQUE, INC.) was added to PBS and CAGE respectively to prepare solutions having a concentration of 125 ng/mL. PBS (pH 7.3) containing 40 µM 4MU-Neu5Ac was added to the mixed solution of AUSA and PBS (125 ng/mL: AUSA in PBS) and the mixed solution of AUSA and CAGE (125 ng/mL: AUSA in CAGE), and incubated at 37°C for 1 hour. After terminating the reaction, the fluorescence intensity of 4MU released by the reaction to the sialidase was measured using a plate reader thereby to obtain the sialidase activity (Ex/Em = 355 nm/460 nm). The results are shown in Figure 6(a). The results revealed that even when the sialidase was mixed with CAGE (AUSA in CAGE), the sialidase activity was not lost but retained.

Dorsal hair on the left and right of a Wistar male rat (12 weeks of age, Japan SLC, Inc.) was removed to provide sites to which the sample solution was applied. Using the contents shown in the following Table 2, each sample solution was applied to the circular area having a diameter of 15 mm at which rat's hair was removed. The application was performed twice a day for 5 days (total 10 applications).

**[Table 2]**

| Test group | Applied matter |
|---|---|
| PBS | 60 µL of PBS was applied |
| CAGE | 60 µL of CAGE was applied |
| CAGE+AUSA | 60 µL of mixed solution of AUSA and CAGE (AUSA: 1 U/mL) was applied |

On the following day from the last application, the skin tissues to which the sample solution was applied were collected, and a part of the collected skin tissues was homogenized. To the homogenate, PBS (pH 7.3) containing 40 µM 4MU-Neu5Ac was added and incubated at 37°C for 1 hour. After terminating the reaction, the fluorescence intensity of 4MU released by the reaction to the sialidase was measured using a plate reader thereby to obtain the sialidase activity (Ex/Em = 355 nm/460 nm). The results are shown in the graph of Figure 6(b). Further, an elastin level in the homogenate of the collected skin tissues was measured using an elastin colorimetric kit (Fastin Elastin Assay Kit, a product of Biocolor Ltd.). The results are shown in the graph of Figure 6(c).

As shown in Figure 6(b) and Figure 6(c), it was revealed that the application of the sample solution in which CAGE and the bacterial sialidase were mixed (CAGE + AUSA) to the skin increased the sialidase activity in the skin tissues and increased the elastin level in the skin tissues. This revealed that the transdermal application of the bacterial sialidase promoted elastin production.

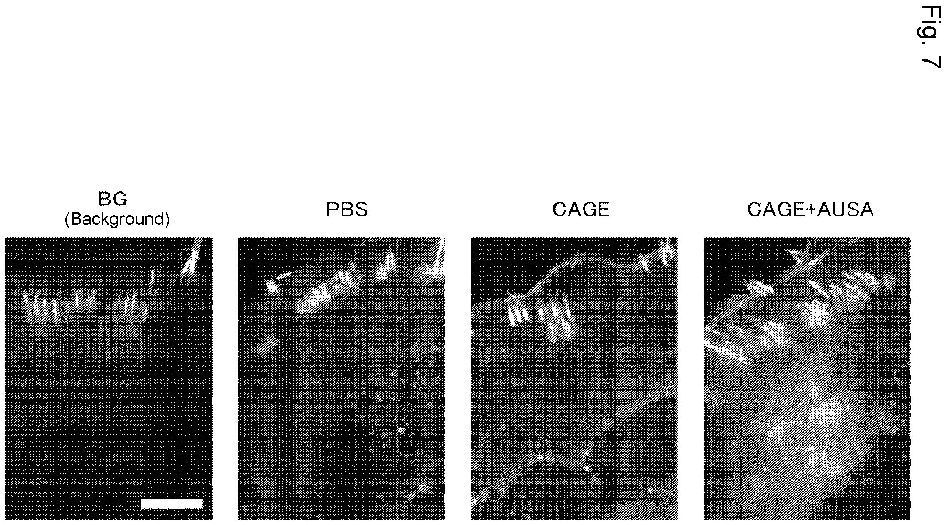

Sites where sialidase activity was present in skin tissues subjected to the application test are shown by a histochemical fluorescence imaging using the fluorescence imaging probe BTP3-Neu5Ac. Specifically, a part of the collected skin tissues was embedded using an embedding medium for frozen tissue section preparation (Tissue-Tek (registered trademark) O.C.T. Compound, Sakura Finetek Japan Co., Ltd.) to freeze tissues, and then a frozen tissue section having a thickness of 100 µm was prepared in a cryostat (Leica Microsystems). After washing with PBS, 150 µL of 300 µM BTP3-Neu5Ac was added and the tissue section was incubated at room temperature (27°C) for 30 minutes. After washing with PBS, the tissue section was observed using a fluorescence microscope (Ex, Em = 360/40, 525/50). The results are shown in Figure 7.

According to the results, more intense fluorescence was observed inside the skin tissues of the skin tissues to which the mixed solution of CAGE and the bacterial sialidase (CAGE + AUSA) was transdermally administered than the skin tissues to which PBS alone or CAGE alone was transdermally administered. This verified that the transdermal administration of the mixed solution of CAGE and the bacterial sialidase delivered the sialidase into the skin tissues and increased the sialidase activity.

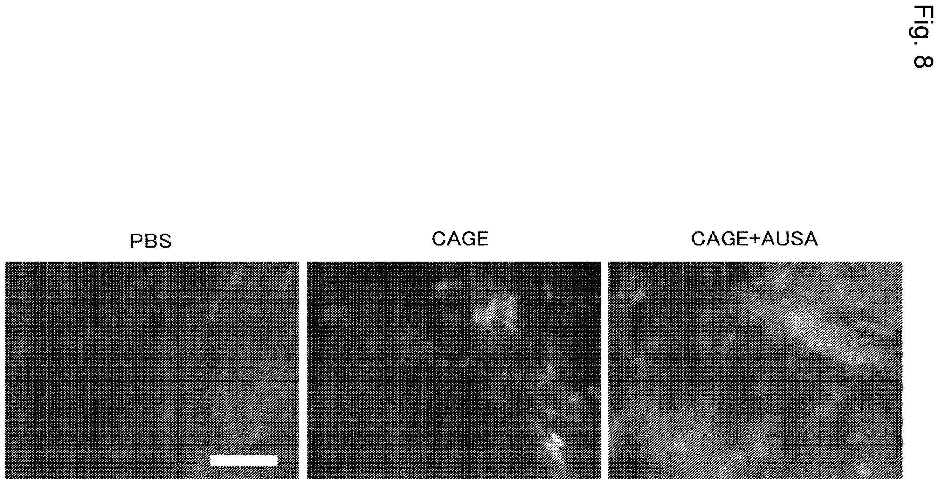

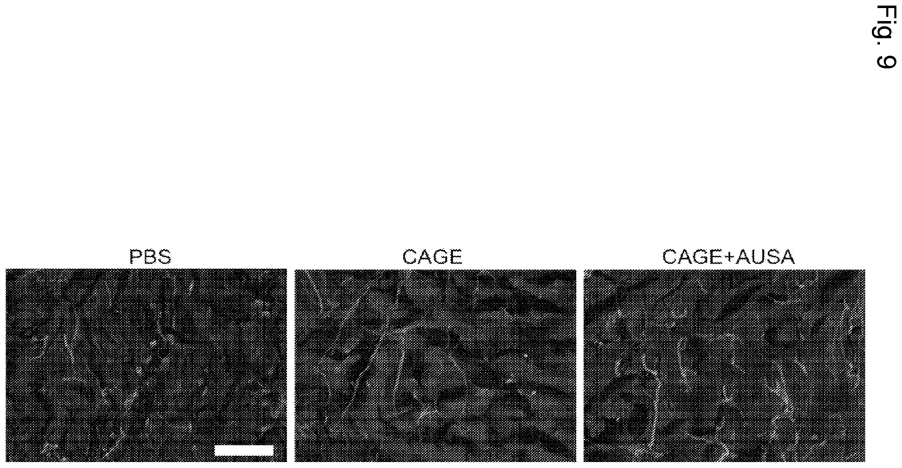

Further, the elastin distribution in the skin tissues subjected to the application test was verified by immunohistostaining (Figure 8) and elastin autofluorescence (Figure 9). Specifically, a flattened section was prepared from the dermis of the collected skin tissues and fixed with 4% paraformaldehyde in PBS. 100 µL of goat serum (Goat Serum 2%) was added as a blocking agent for the immunohistostaining and the section was incubated at room temperature for 30 minutes. Subsequently, 100 µL of a primary antibody (Elastin Polyclonal antibody) was added and incubated at 4°C overnight. Then, 100 µL of a secondary antibody (Goat Anti-Rabbit IgG) was added and the section was incubated at room temperature for 30 minutes, and then observed using a microscope (Ex/Em = 470 nm/525 nm). The results of immunohistostaining are shown in Figure 8.

According to the results, stained elastin was observed in a wide area inside the skin tissues of the skin tissues to which the mixed solution of CAGE and the bacterial sialidase (CAGE + AUSA) was transdermally administered than the skin tissues to which PBS alone or CAGE alone was transdermally administered. This revealed that the transdermal administration of the mixed solution of CAGE and the bacterial sialidase delivered the sialidase into the skin tissues and promoted elastin production in the skin tissues.

On the other hand, as elastin is an autofluorescent substance that emits autofluorescence, the dermis section fixed with 4% paraformaldehyde in PBS was observed for the elastin autofluorescence using a fluorescence microscope. The results are shown in Figure 9. In the skin tissues to which the mixed solution of the bacterial sialidase and CAGE (CAGE + AUSA) was applied, abundant elastin autofluorescence was observed and an increased elastin level was observed.

### [Example 6]

### 6. Elastin production promoting effect on senescence-accelerated model mice

In the present Example, an investigation was made, using senescence-accelerated model mice, whether elastin production can be promoted by sialidase transdermal administration to individuals whose sialidase activity was reduced and elastin level was decreased with age.

For the senescence-accelerated model mice, SAMP1 and SAMP8 in which SAMP (Senescence-Accelerated Mouse Prone) indicating senescence accelerated or short lived were selected (24 weeks of age, male, Japan SLC, Inc.). Dorsal hair on the left and right of a senescence-accelerated model mouse was removed to provide sites to which the sample solution was applied. Using the contents shown in the following Table 3, each sample solution was applied to the circular area having a diameter of 10 mm at which mouse's hair was removed. The application was performed twice a day for 5 days (total 10 applications).

**[Table 3]**

| Test group | Applied matter |
|---|---|
| PBS | 40 µL of PBS was applied |
| CAGE+AUSA | 40 µL of mixed solution of AUSA and CAGE (AUSA: 1 U/mL) was applied |

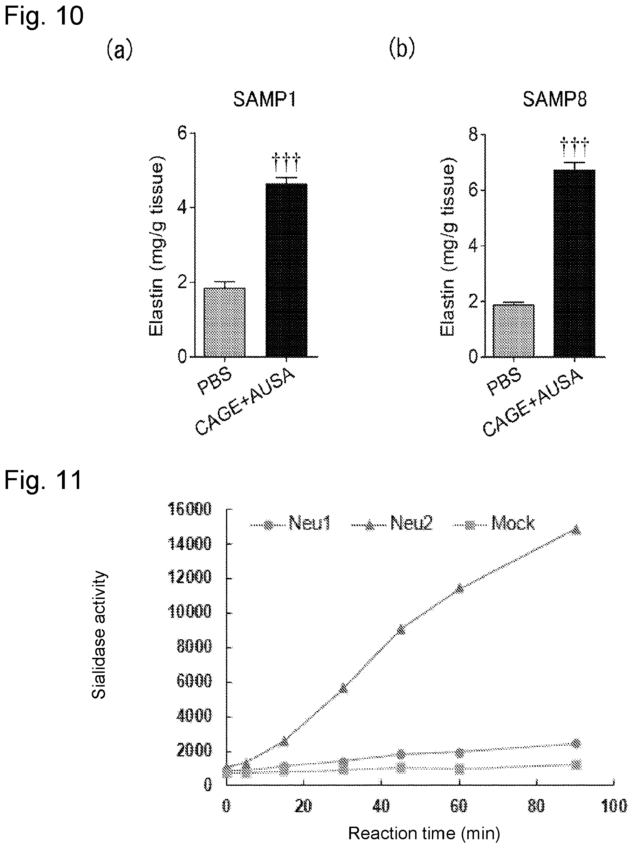

On the following day from the last application, the skin tissues to which the sample solution was applied were collected, and a part of the collected skin tissues was homogenized. An elastin colorimetric kit (Fastin Elastin Assay Kit, a product of Biocolor Ltd.) was used on the homogenate to measure an elastin level in the homogenate of the collected skin tissues. The results are shown in the graph of Figure 10.

The results revealed that the sialidase transdermal administration to the senescence-accelerated model mice increased the elastin expression level. Thus, it was revealed that the sialidase transdermal administration promoted elastin production in the dermis and increased an elastin level in the dermis by transdermally delivering the sialidase even in the condition where an elastin level has been decreased with aging, thereby having an anti-aging effect.

### [Example 7]

### 7. Study on stability of the sialidase enzyme activity

NEU1 Producing cells which produce rat-derived sialidase NEU1 were obtained in the same manner as in Example 4 and using C6 glioma cells. The NEU1 producing cells were cultured in MEM medium to express sialidase NEU1. The cells were recovered and lysed thereby to obtain a lysate containing the expressed sialidase NEU1. To the lysate, PBS (pH 4.6) containing 4MU-Neu5Ac was added and reacted at 27°C for a predetermined period of time. As the optimum pH of sialidase NEU1 is in the acid region of 4.6, pH of the reaction solution is 4.6. After terminating the reaction, the fluorescence intensity of 4MU released by the reaction to the sialidase was measured using a plate reader thereby to quantitatively determine the sialidase activity of sialidase NEU1 in the lysate (Ex/Em = 355 nm/460 nm). Additionally, the same test was also performed on the NEU2 producing cells that produce the rat-derived sialidase NEU2 obtained in Example 4 thereby to quantitatively determine the sialidase activities of sialidase NEU2 in the lysate at predetermined reaction times. As the optimum pH of sialidase NEU2 is in the neutral region, pH of the reaction solution is 7.3 as in Example 4. The results are shown in Figure 11. Mock in Figure 11 shows data of the empty vector case where no insert DNA fragment is incorporated.

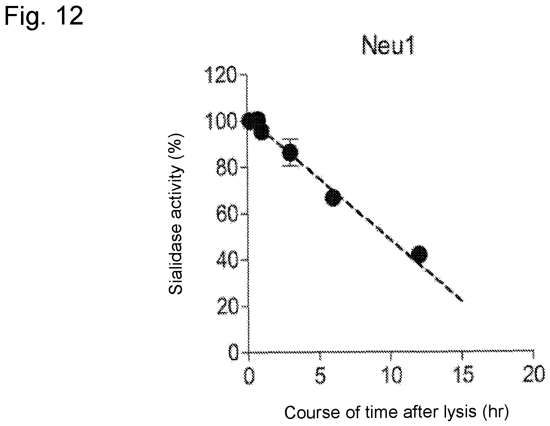

Further, the lysate containing sialidase NEU1 described above was quantitatively determined for sialidase activity in the course of time after lysis to investigate the stability of enzyme activity. PBS (pH 4.6) containing 4MU-Neu5Ac was added to the lysate after a predetermined time had passed from the lysis treatment and reacted at 27°C for 1 hour. After terminating the reaction, the fluorescence intensity of 4MU released by the reaction to the sialidase was measured using a plate reader thereby to quantitatively determine sialidase activity. The results are shown in Figure 12.

The results showed that sialidase NEU2 obtained the higher sialidase activity than sialidase NEU1 (Figure 11). The results also showed that the enzyme activity of sialidase NEU1 quickly diminished after the cells were lysed (Figure 12). The results revealed that sialidase NEU2, compared to sialidase NEU1, can be more suitably used for the application to the skin as the elastin production promoter.

The present invention is not limited to the above embodiments or Examples, but encompasses various modified design forms in the technical scopes within the range of not deviating from the summary of invention described in the scope of claims for patent.

### Industrial Applicability

The present invention provides the elastin production promoter and the cosmetic preparation for skin with an excellent elastin production promoting effect and capable of preventing or ameliorating the decrease of dermal elastin with aging, and thus is widely useful in the industries such as cosmetic preparations field, and pharmaceutical products and quasi-drugs fields.

## Claims

1. An elastin production promoter comprising a sialidase as an active ingredient.

2. The elastin production promoter according to claim 1, wherein the sialidase is sialidase NEU2.

3. The elastin production promoter according to claim 1, wherein the sialidase is a bacterial sialidase.

4. The elastin production promoter according to claim 3, wherein the bacterial sialidase is a sialidase derived from a bacterium of the genus *Arthrobacter (Arthrobacter* sp.).

5. The elastin production promoter according to any one of claims 1 to 4, wherein the elastin is dermal elastin constituting fibrous tissues of the dermis.

6. The elastin production promoter according to any one of claims 1 to 5, wherein the elastin production promoter is for ameliorating or preventing skin aging.

7. A cosmetic preparation for skin, comprising the elastin production promoter according to any one of claims 1 to 6.
